# EUROPEAN PATENT APPLICATION

(11) **EP 0 537 383 A1**
(43) Date of publication of application: **21.04.1993**
(21) Application number: 91202676.2
(22) Date of filing: 15.10.1991
(51) Int. Cl.: A61B 5/024, A61B 5/025

(54) **Inflatable finger cuff for use in non-invasive monitoring of instaneous blood pressure**

(71) Applicant: NEDERLANDSE ORGANISATIE VOOR TOEGEPAST-NATUURWETENSCHAPPELIJK ONDERZOEK TNO, NL-2628 VK Delft (NL)
(72) Inventor: Hyndman, Barry William, NL-1015 BD Amsterdam (NL)
(74) Representative: de Bruijn, Leendert C.

(57) **Abstract**

An inflatable finger cuff for encircling the finger of a patient, to be used for non-invasive continuous monitoring of the instantaneous arterial blood pressure of the patient. Said finger cuff comprises a rigid cylinder (2), a compliant thin-walled tube (3) insertable within the housing and forming an inflatable cylinder-like space therewith, said tube in use encircling said finger, the cylinder being pneumatically connectable to a fluid source (11), and an infrared light source (5) and infrared light detector (6) electrically connectable to an outside control circuit and being positioned in substance on both sides of the finger opposite each other. Said light source and light detector are located within said inflatable space between the rigid cylinder and the compliant tube, which is made of translucent material, are fastened to the inner wall of the compliant tube by an adhesive. A method of making an inflatable cuff adapted to encircle a finger of a patient, said method including the steps of providing a rigid conically shaped cylinder, having a first nozzle (7) by way of a side connection for pressurization by a gas, a second nozzle by way of another side connection for passing electrical wires (10) for the light elements, inserting a compliant thin-walled translucent tube, and soldering the light elements to the wires. Furthermore said method includes the step of fastening the light elements to the inner wall of the compliant tube by means of an adhesive, rolling the compliant tube back over the cylinder at the ends and fastening the tube to said ends to form a gastight seal.

## Description

The invention relates to an inflatable finger cuff for encircling the finger of a patient, to be used for non-invasive monitoring of the instantaneous arterial blood pressure of the patient, said finger cuff comprising a rigid cylinder, a compliant thin-walled tube insertable within the housing and forming an inflatable cylinder-like space therewith, said tube in use encircling said finger, the cylinder being pneumatically connectable to a fluid source, and an infrared light source and infrared light detector electrically connectable to an outside control circuit, which pair of light source and light detector is to be positioned in substance on both sides of the finger opposite each other. Such an inflatable finger cuff is known from "Medical & Biological Engineering & Computing" Vol. 20, May 1982, pp. 314-318 by Yahmakoshi et al and Vol. 20, January 1991, pp. 55-62 by Kawarada et al.

In the inflatable finger cuff described in above periodicals, the light source and light detector are fixed directly on the skin of the finger, for instance, by a piece of adhesive tape. The rigid cylinder of the cuff, into which a thin-walled rubber tube is inserted and fixed at its flanges at both ends by a pair of acrylic caps, is then placed over the light source and detector pair. The wires to both the light source and light detector under adhesive tape are led out along the finger. The cuff is pneumatically connected to the rest of the blood pressure system by way of a side connection of the rigid cylinder.

When such a finger cuff is pressurized to approximately mean arterial pressure level, there appears a slow upward drift in the mean level of the light detector signal or "plethysmogram" over a period of minutes. Such a drift has a detrimental effect on the accuracy of the blood pressure measurement since the original reference value, to which the arteries are clamped and which is determined by the processing circuit on the basis of the first 30 seconds of plethysmogram signal, will no longer be valid and the monitor will underestimate the pressure. It is possible to use a recalibrate algorithm such that, after a period of f.i. two minutes, the 'fine scanning phase' of the initialization or reference determining algorithm is restarted. Then the cuff pressure starts not at zero level, around which most of the drift takes place, but at a value some 20 mmHg below the current average pressure after most of the drift has occurred.

The above mentioned slow drift is most probably a result of the light source and/or detector with the connecting wires being slowly pressed into the finger tissue by the cuff pressure underneath the thin-walled compliant tube. The consequence is that more light from the light source gradually reaches the light detector.

The invention aims to obviate above mentioned problem and to provide an inflatable finger cuff in which initial or later drift in the plethysmogram is no longer present and in which the reference level determined is much more stable.

This objective is attained in an inflatable finger cuff of the above mentioned type according to the invention in that the light source and light detector are located within said inflatable space between the rigid cylinder and the compliant tube, which tube is made of translucent material.

With the above mentioned finger cuff the plethysmogram responds to sudden pressurization to approximately mean arterial pressure quickly and the signal stabilizes with no sign of drift. This is directly the result of the fact that the light source and light detector cells together with the connecting wires are located inside the pressurized space of the cuff and are no longer impressed into the tissue of the finger. Another advantage is the fact that the inconvenience of first positioning the light cells on the finger and afterwards placing the finger cuff over the cells, is obviated.

Another finger cuff is disclosed in EP-A-0260807. This European patent application describes a finger cuff comprising an inflatable urethane bladder adapted to encircle the finger. Such a flexible finger cuff has the advantage of fitting a wide range of finger sizes. However, the infrared light source and detector cells are mounted in the inflatable bladder in through-going openings, into which the cells are placed, at points where the inner and outer walls of the bladder have been heat-sealed to each other. In the region of and surrounding these openings, where the outer and inner wall of the bladder come together surrounding the cells in these openings, only a portion of the pressure in the bladder is transmitted to the arteries underneath the bladder, precisely at the location where arterial cross-section is being monitored by the cells and thus where 100% pressure transfer should take place. Therefore inadequate pressure transfer to the arterial walls results. An other disadvantage of this flexible construction is that the measured pressure is influenced by how tightly said flexible cuff is wrapped around the finger.

The invention will now be explained on the basis of an exemplary embodiment with reference to the drawings, in which:
Figure 1 shows the light detector signal or plethysmogram of a finger cuff of the state of the art against cuff pressurization to approximately mean arterial pressure;
Figure 2 shows a diagrammatic view of the finger cuff according to the invention;
Figure 3 shows the light detector signal or plethysmogram of the finger cuff of figure 2 against cuff pressurization to approximately mean arterial pressure;
Figure 4 shows a comparison between measurements on the finger cuff of figure 2 and the finger cuff according to the state of the art;
Figure 5 shows measurement values of systolic pressure of the finger cuff of figure 2 against occlusion cuff pressure; and
Figure 6 shows the plethysmogram of the finger cuff of figure 2 with successive determinations of the reference level by the recalibrate function.

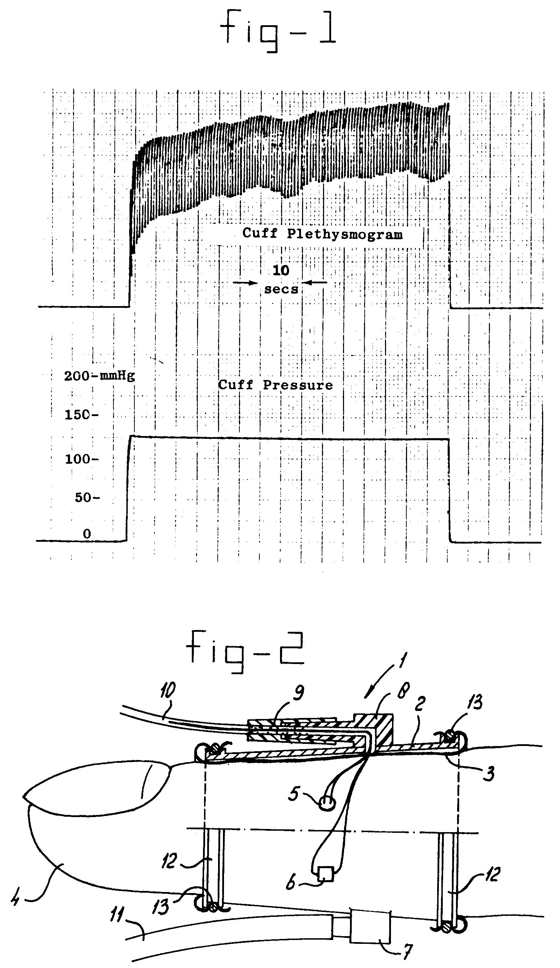

In the state of the art, the cuff is pneumatically connected to the blood pressure system by way of a side connection on the rigid cylinder. When the finger cuff is pressurized to approximately mean arterial pressure, the cardiac pulsation, as indicated in figure 1, is clearly evident and there is a slow upward drift in the mean level of the plethysmogram, often over a period of several minutes. The slow drift, as explained before, is due to the fact that the light source and light detector cells are slowly pressed into the finger tissue by the cuff pressure underneath the thin-walled rubber tube. The consequence is that more light from the light source will gradually reach the detector cell. This will have a detrimental effect on the accuracy of the blood pressure measuring system since the original reference value, to which the arteries are clamped and which is determined by the system processing circuit on the basis of the first 30 seconds of plethysmogram signal, would, after several minutes, be no longer correct. The monitor would then underestimate the pressure.

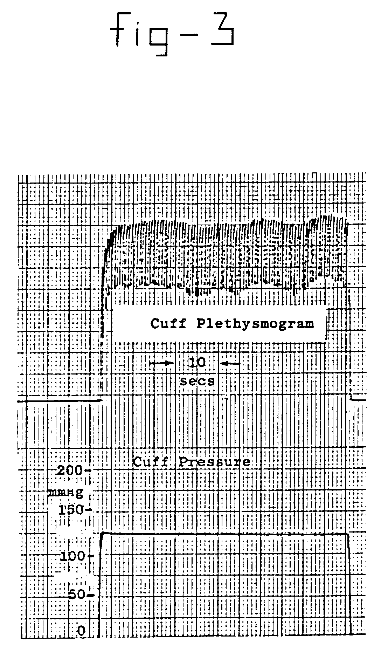

Now in Figure 2 the finger cuff according to the invention is shown diagrammatically. Such finger cuff 1 is to be placed around a finger generally indicated with 4. The cuff comprises a conically shaped rigid cylinder 2 into which a thin-walled conical tubing 3 of translucent material is inserted. The light source and light detector cells 5 and 6, respectively, are located within the space to be pressurized between tube 3 and rigid cylinder 2. Said photocells are soldered to the wires and fastened to the thin-walled tube by means of an adhesive, like rubber cement. The translucent tube is then rolled back over the cylinder 2 at both ends where it is held in place by O-rings 13 fitted over grooves 12 on the cylinder ends tight enough to form a gas or fluidum-tight seal. Preferably gas, e.g. air, is used as medium for pressurization. There are two side connections on the cylinder, one nozzle 8 with a plastic tubing 9 for the electrical cable 10 comprising the wiring for the light cells 5 and 6 and one nozzle 7 for a plastic air tubing 11 for pressurization. The connection of 8, 9 and 10 is made airtight by injecting silicon into the tubing and nozzle.

Figure 3 shows the manner in which the light detector signal or plethysmogram in the finger cuff according to the invention responds to sudden pressurization to approximately mean arterial pressure. With the appearance of the cardiac pulse, the plethysmogram signal quickly stabilizes (viz., within five seconds). There is no sign of drift contrary to the construction of the known finger cuff in which the cells are placed underneath the thin-walled rubber tube onto the finger skin.

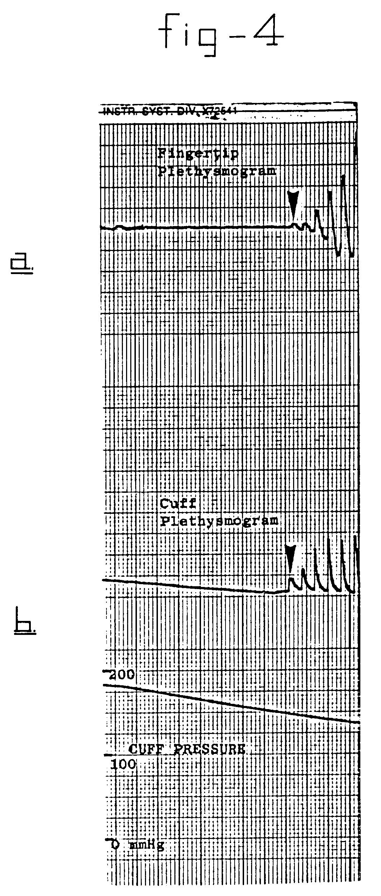

Figure 4 shows a comparison of the known finger cuff and the finger cuff according to the invention. In Figure 4A the plethysmogram is shown of the known finger cuff placed on the ring finger of one hand, in which arrangement the light source and detector cells are attached to the finger skin, here, on the fingertip, distal to the cuff. Such an arrangement is called an 'occlusion cuff' and is known to accurately measure systolic blood pressure in the finger. Figure 4B shows the plethysmogram of a finger cuff according to the invention placed on the middle finger of said same hand. The cuffs are pneumatically mutually connected to each other and are inflated suprasystolically and are then allowed to slowly deflate. It can be seen that a cardiac pulse appears simultaneously from both cuffs, thus demonstrating that the new cuff can accurately measure systolic pressure in the finger.

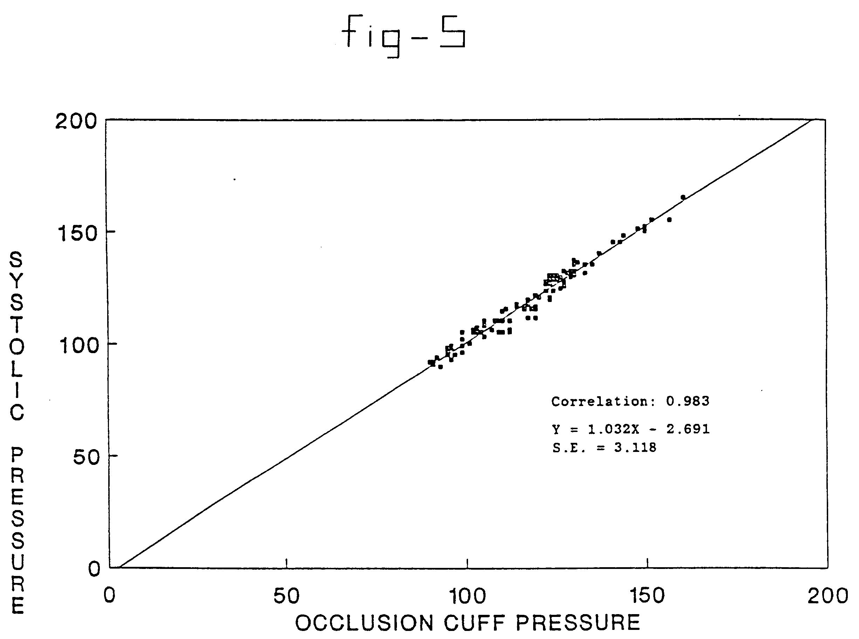

Figure 5 shows a scatterplot of the measurement results of systolic blood pressure obtained from occlusion cuff determinations, in the manner described above, and corresponding systolic pressures obtained from the noninvasive continuous blood pressure monitoring system such as disclosed in United States Patent 4,475,554 and the present new finger cuff. The results show that an error of only 3 mmHg is obtainable. From a theoretical standpoint this is not surprising since the only material which stands between the pressure in the cuff and the finger per se is a thin compliant translucent membrane or tube.

Now, in every continuous blood monitoring system based on an inflatable finger cuff, a 'recalibrate' algorithm is used to intermittently correct for drift in the plethysmogram signal since the last determination of the reference value. The feedback loop in the control circuit which clamps the arterial cross section to its unloaded value (the 'reference' value) is opened, the cuff pressure is swept, and the new reference value is determined from the plethysmogram signal and the loop is again closed.

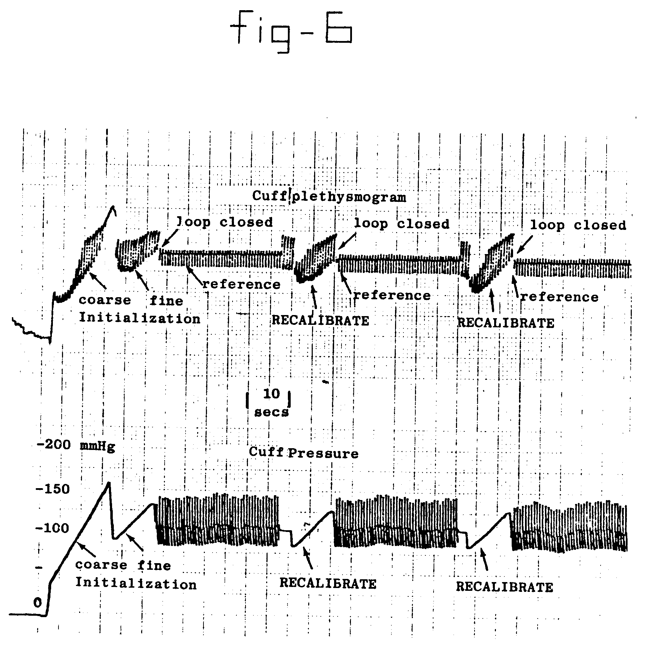

Figure 6 shows that, with the present finger cuff, repeated determinations of the reference level yield virtually identical reference values, indicating that drift has largely been eliminated.

## Claims

1. An inflatable finger cuff for encircling the finger of a patient, to be used for non-invasive continuous monitoring of the instantaneous arterial blood pressure of the patient, said finger cuff comprising a rigid cylinder, a compliant thin-walled tube insertable within the housing and forming an inflatable cylinder-like space therewith, said tube in use encircling said finger, the cylinder being pneumatically connectable to a fluid source, and an infrared light source and infrared light detector electrically connectable to an outside control circuit, which pair of light source and light detector is to be positioned in substance on both sides of the finger opposite each other, characterized in that the light source and light detector are located within said inflatable space between the rigid cylinder and the compliant tube, which is made of translucent material.

2. An inflatable finger cuff according to claim 1, wherein the light source and light detector are fastened to the inner wall of the compliant tube by an adhesive.

3. An inflatable finger cuff according to claim 2, wherein the adhesive is rubber cement.

4. A method of making an inflatable cuff according to one of the preceding claims adapted to encircle a finger of a patient, said method including the steps of providing a rigid conically shaped cylinder, having a first nozzle by way of a side connection for pressurization by a gas, a second nozzle by way of another side connection for passing electrical wires for the light elements, inserting a compliant thin-walled translucent tube, and soldering the light elements to the wires, characterized by the step of fastening the light elements to the inner wall of the compliant tube by means of an adhesive, and by rolling the compliant tube back over the cylinder at the ends and fastening the tube to said ends to form a gastight seal.
